# EUROPÄISCHE PATENTSCHRIFT

(11) **EP 1 549 421 B1**
(45) Veröffentlichungstag und Bekanntmachung des Hinweises auf die Patenterteilung: **13.06.2012**
(21) Anmeldenummer: 03807830.9
(22) Anmeldetag: 01.10.2003
(51) Int. Cl.: C12M 1/26, G01N 1/14

(54) **VORRICHTUNG UND VERFAHREN ZUR ENTNAHME VON FLÜSSIGEN PROBEN**
DEVICE AND METHOD FOR EXTRACTING LIQUID SAMPLES
DISPOSITIF ET PROCÉDÉ DE PRÉLÈVEMENT D'ÉCHANTILLONS LIQUIDES

(30) Priorität: 02.10.2002 DE 10246262
(43) Veröffentlichungstag der Anmeldung: 06.07.2005
(73) Patentinhaber: Max-Planck-Gesellschaft zur Förderung der Wissenschaften e.V., 80539 München (DE)
(72) Erfinder: SANN, Heiner, 39104 Magdeburg (DE); BOCK, Andreas, 39114 Magdeburg (DE); REICHL, Udo, 30938 Burgwedel (DE)
(74) Vertreter: Hannke, Christian
(86) Internationale Anmeldenummer: PCT/EP2003/010883
(87) Internationale Veröffentlichungsnummer: WO 2004/033077

(56) Entgegenhaltungen:
- DE-C- 19 530 886
- FR-A- 2 617 286
- FR-A- 2 647 213
- US-A- 4 501 161
- PATENT ABSTRACTS OF JAPAN Bd. 0183, Nr. 67 (C-1223), 11. Juli 1994 (1994-07-11) & JP 06 098758 A (AGENCY OF IND SCIENCE & TECHNOL; OTHERS), 12. April 1994 (1994-04-12)
- PATENT ABSTRACTS OF JAPAN Bd. 0100, Nr. 84 (P-442), 3. April 1986 (1986-04-03) & JP 60 219539 A (RIKAGAKU KENKYUSHO;OTHERS), 2. November 1985 (1985-11-02)

## Beschreibung

Die Erfindung betrifft eine Vorrichtung und ein Verfahren zur Entnahme von flüssigen Proben aus mit einem Medium gefüllten Behältern und/oder Rohren, insbesondere aus Fermentern, über eine Filtermembran mittels Unterdruck gemäß den Oberbegriffen der Patentansprüche 1 und 15.

Eine Vorrichtung zur Entnahme von flüssigen Proben aus einem Fermenter ist aus der DE 32 49 399 T1 bekannt. Für die Entnahme der Probe weist die Vorrichtung einen Probenehmer in Form eines normal geschlossenen Ventils auf, an dessen Gehäuse auf der Seite eines Kontaktes mit einem Probeaufnehmer ein ringförmiger Vorsprung ausgebildet ist, der zur Aufnahme einer Ringnut eines Gehäuses eines normal geschlossenen Ventils des Probeaufnehmers vorgesehen ist. Zwischen dem normal geschlossenen Ventil des Probeaufnehmers und einem Halt des Probeaufnehmers ist ein bakteriendichter Filter angeordnet, der zu dem normal geschlossenen Ventil des Probenehmers beabstandet ist. Diese Beabstandung hat zur Folge, dass bei der Entnahme einer Probe ein dadurch entstandener Zwischenraum innerhalb der Vorrichtung ebenso mit der Probeflüssigkeit gefüllt werden muß, jedoch nicht als zu überprüfende Probeflüssigkeit in den Probeaufnehmer mit aufgenommen wird. Ein dadurch enstandenes Totvolumen der entnommenen Probe bewirkt, dass zum einen die Probenahme nicht sparsam in Bezug auf das benötigte Probevolumen pro Probeentnahme durchgeführt werden kann, und zum anderen ein Restvolumen aus der vorangegangenen Probeentnahme innerhalb der Vorrichtung zurückgeblieben ist, das aufgrund seiner nicht-sterilen Eigenschaften zu einer Verfälschung der Prüfergebnisse der nachfolgend durchgeführten Probeentnahme mit gewünschter Sterilität führen kann.

Bekannt sind auch kommerziell erhältliche Probenahmevorrichtungen zur Entnahme von Proben einer in einem Bioreaktor mit einem beispielsweise 5-Liter-Volumen durchgeführten Fermentation, die stabförmig ausgebildet sind und an deren Ausgang sich ein Drei-Wege-Ventil befindet, wie es beispielsweise in der US 5,948,998 gezeigt wird. Das Drei-Wege-Ventil dient zur Umschaltung des Strömungsverlaufs während der Probenahme von dem Weg zwischen der Probenahmesonde und dem Ausgang auf einen Weg zwischen einem Überströmablauf und dem Ausgang. Eine derartige Umschaltung der Strömungswege findet statt, nachdem eine Probe mit wunschgemäßem Volumen in ein der Probenahmesonde nachfolgendes Leitungssystem gefördert wurde.

Aufgrund dieses Umschaltvorganges reißt der innerhalb der Vorrichtung bestehende Flüssigkeitsstrom ab, wodurch eine verstärkte Durchmischung der Probeströmung erhalten wird. In diesem Fall führt die höhere Durchmischung zur Vergrößerung des benötigten Probevolumens oder der Anzahl der zu entnehmenden Proben bis zur Einstellung einer konstanten Konzentration der Substanzen innerhalb der Probe, da eine Rückvermischung einzelner Substanzen aufgrund einer nicht idealen Verdrängung der Probenflüssigkeit aus der Vorrichtung aus der vormals durchgeführten Probenahme stattfindet. Da derartige Probenahmevorrichtungen ein Totvolumen aufweisen, ist die restliche Probeflüssigkeit der vorangegangenen Probenahme nicht vollständig aus der Vorrichtung verdrängbar, wodurch aufgrund der fluiddynamischen Eigenschaften der Probeflüssigkeit eine Rückvermischung stattfindet.

Zudem findet eine Rückvermischung auch aufgrund unterschiedlicher Diffusionskoeffizienten der in der Probe enthaltenen Substanzen statt.

FR 2 617 286 A offenbart eine Vorrichtung zur sterilen Entnahme von Flüssigkeiten aus einem Behälter wie einem Bioreaktor durch eine Filtermembran. Mit dieser Vorrichtung ist es durch Ventilsteuerung möglich, Gase wie sterile Druckluft oder Wasserdampf durch die Zuleitung zur sterilen Grenzseite der Filtermembran zu befördern. Ebenfalls ist es durch Ventilsteuerung und Erzeugung eines Unterdrucks mittels Pumpe möglich, eine sterilgefilterte Probe durch Leitungen bis zu einem Probenraum zu befördern.

JP 60 219539 A beschreibt ebenfalls ein Verfahren und eine Vorrichtungen zum Entnehmen von gefilterten Proben. Der Transport der entnommenen Probe bis zu einem Probenraum erfolgt durch mittels eines Pumpensystems angelegten Unterdruck. Neben einer Leitung zum Probenehmen und Gaszuführen ist eine weitere Leitung vorgesehen, um Hilfs-Unterdruck für die Probenahme zu erzeugen.

DE 195 30 886 C1 betrifft eine Probenahmevorrichtung, die fluidische Dioden zur tropfenweisen Dosierung der zu entnehmenden Probe und einer Kalibrierlösung ermöglicht. Die fluidischen Dioden sind zwischen der zu dem Medium hingeordneten Filtermembran und einem Sensor zur Detektion bestimmter Prüfdaten angeordnet. Da die fluidischen Dioden selbst kleine Abmessungen aufweisen, ist ein Totraum zwischen der Filtermembran und dem Sensor minimiert.

Derartige Probenahmevorrichtungen weisen ebenso ein Totvolumen innerhalb des Totraums bei der Probenahme auf, das zum einen eine Probenahme mit höherem Volumen erfordert, und zum anderen die Gefahr einer Vermischung der in dem Totvolumen angeordneten Restprobe aus der vorangegangenen Probenahme mit den Probesubstanzen der neuen Probenahme aufwirft.

Die vorgenannten Vorrichtungen lassen eine Überprüfung der Unversehrtheit der zu dem Medium hin angeordneten Filtermembran nur bedingt oder mit großem Aufwand zu, wie beispielsweise den Ausbau der Filtermembran. Eine Undichtigkeit der Filtermembran hat zur Folge, dass die Sterilgrenze des Behälters oder der Rohrleitung durchbrochen und das Medium unsteril wird.

Desweiteren ist eine Überprüfung der Dichtigkeit der Filtermembran lediglich vor dem Einbau und nach dem Ausbau einer Probeentnahmevorrichtung möglich, so dass immer erst das Ende des gesamten Prozesses oder Messzyklus abzuwarten ist, bevor ein derartiger Integritätstest durchgeführt werden kann.

Demzufolge liegt der vorliegenden Erfindung die Aufgabe zugrunde, eine Vorrichtung und ein Verfahren zur Entnahme von flüssigen Proben aus mit einem Medium gefüllten Behältern und/oder Rohren über eine Filtermembran zur Verfügung zu stellen, bei der/dem eine Entnahme der Probe ohne Totvolumen unter zuverlässig sterilen Bedingungen, die mittels eines Integritätstests jederzeit überprüft werden können, möglich ist.

Diese Aufgabe wird vorrichtungsseitig durch die Merkmale des Patentanspruches 1 und verfahrensseitig durch die Merkmale des Patentanspruches 15 gelöst.

Kerngedanke der Erfindung ist es, bei einer Vorrichtung zur Entnahme von flüssigen Proben aus mit einem Medium gefüllten Behältern und/oder Rohren, insbesondere aus Fermentern, über eine Filtermembran mittels Unterdruck, der z. B. durch eine Pumpe erzeugt werden kann, die innerhalb einer Probensonde angeordnete Filtermembran aus einem als Sterilgrenze wirkenden Material anzufertigen, wobei auf der dem Medium abgewandten sterilen Grenzseite der Filtermembran eine gasführbare Zuführleitung und eine separate probeführbare Abführleitung angeordnet sind. Die probeführbare Abführleitung ist mit der Pumpe zur Bildung eines Unterdrucks, aufgrund dessen die Probe durch die Filtermembran hindurch aus dem Medium angesaugt wird, verbunden.

Nach erreichter Entnahme einer Probe mit einem vorbestimmten Probevolumen, welches z. B. mittels der Pumpe reguliert wird, wird die Probe mit zusätzlichem geringem Gasüberdruck, das einer zur Vorrichtung hingewandten Rückseite der Filtermembran mittels der Zufuhrleitung zugeführt wird, über die Abführleitung aus dem Leitungssystem der Probenahmevorrichtung abgeführt. Gleichzeitig wird das Leitungssystem zuverlässig mittels des zugeführten Gases entleert. Auf diese Weise ist kein Totraum innerhalb der Probenahmevorrichtung für die Bildung eines Totvolumens vorhanden. Somit ist eine Entnahme der Probe mit dem gewünschten zu überprüfenden Probevolumen selbst bei kleinen Volumenmengen möglich, ohne dass zusätzliches Probevolumen für das Zur-Verfügung-Stellen eines Totvolumens notwendig ist.

Da die Filtermembran mit dem als Sterilgrenze wirkenden Material ausgestattet ist, wird die Entnahme einer sterilen Probe, unter der Voraussetzung, dass das Leitungssystem und die restlichen Einrichtungen der Probenahmevorrichtung in sich steril sind, sichergestellt.

Selbst wenn die Membran eine Undichtigkeit aufweist, ist eine sterile Funktion der Probenahmevorrichtung sichergestellt, vorausgesetzt, dass der aufgrund der Undichtigkeit entstehende Druckabfall im Leitungssystem während des Probenahmevorganges fortlaufend durch das zuzuführende Gas ausgeglichen wird. Es kann zwar aufgrund der defekten Filtermembran etwas Spülflüssigkeit zum wahlweisen Ausspülen des Leitungssystems in das Medium gelangen, jedoch beeinflusst die Verdünnung des Mediums durch die Spülflüssigkeit, die beispielsweise destilliertes Wasser sein kann, die Konzentration der in dem Medium enthaltenen Substanzen nicht nachweisbar.

Das zur Entleerung des Leitungssystems verwendete Gas ist beispielsweise Druckluft, die wie ein hydrophobes Medium wirkt. Demgegenüber ist die Filtermembran aus einem hydrophilen Material zusammengesetzt, das nur bei hohem Überdruck von dem Gas durchdrungen werden kann. Eine durch diese Eigenschaften des Gases und des Filtermembranmaterials hervorgerufene zuverlässige Entleerung des Leitungssystems ohne Durchdringung der Filtermembran in Richtung des Mediums hat eine vollständige Befreiung nicht nur des Leitungssystems, sondern auch der restlichen Vorrichtungseinrichtungen von der Probenflüssigkeit der vorangegangenen Probenahme zur Folge. Somit wird auf einfache Weise eine vollständige Entleerung des Vorrichtungsinnenraumes von Probenflüssigkeit und einer dadurch bedingten totvolumenfreie Probenahme erreicht.

Vorteilhaft kann auch aus nicht-sterilen Medien eine sterilgefilterte Probe entnommen werden, da die Filtermembran als Sterilgrenze dient. Derartigen sterilgefilterten Proben ist eine erhöhte Lagerfähigkeitsdauer zu eigen.

Da eine automatische Entleerung auf einfache und wirkungsvolle Art im Anschluß an die Probenahme stattfindet, ist die erfindungsgemäße Probenahmevorrichtung als Probenahmemodul automatisiert einsetzbar und ermöglicht den Anschluß von Detektionssystemen, die für eine prozessnahe Kontrolle und Regelung/Steuerung der entnommenen Probe und der erfindungsgemäßen Vorrichtung dienen.

Zudem kann der Zeitraum für einen Probenahmevorgang durch geeignete Kombination von dem zu entnehmenden Probevolumen, der durch die Pumpe bedingten Fördergeschwindigkeit und der benötigten Zeitspanne für die Entleerung des Leitungssystems ausreichend kurz für Online-Messungen gehalten werden.

Ein Integritätstest zur Überprüfung der Dichtigkeit der Filtermembran ist zu einem beliebigen Zeitpunkt möglich. Ein derartiger Test kann vor und nach einem Probenahmevorgang durchgeführt werden und ermöglicht demzufolge eine Erhöhung der Betriebssicherheit des Behälters, der einen Bioreaktor darstellen kann, in Verbindung mit der daran befestigten Probenahmevorrichtung und eine Verbesserung der Handhabung der Probenahmevorrichtung.

Für einen Integritätstest, der die Funktion der Probenahmevorrichtung in situ überprüft, ist die gasführbare Zuführleitung mit einer ersten gasführenden Anschlussleitung zum verbinden der Zuführleitung mit einem Gasversorgungsanschluß gekoppelt. Eine derartige Koppelung ist ebenso für die Zuführung des Gases zur Entleerung des Leitungssystems erforderlich.

Ein erstes und zweites Ventil sind im Bereich eines ersten und zweiten Endes der ersten gasführenden Anschlußleitung gemäß einer bevorzugten Ausführungsform angeordnet, so dass die Anschlussleitung beiderseitig mittels des Ventils gesperrt werden kann. Zusätzlich sind ein Drucksensor in der gasführenden Anschlussleitung sowie ein erster Sterilfilter im Bereich des Gasversorgungsanschlusses innerhalb der gasführenden Anschlussleitung angeordnet, so dass der sterile Betrieb des Leitungssystems und insbesondere der gasführenden Anschlussleitung sichergestellt ist und eine Überprüfung des üblicherweise leichten Überdrucks innerhalb der Gasleitung möglich ist.

Gemäß einer bevorzugten Ausführungsform sind die Zu- und Abführleitungen derart ausgebildet, dass sie zum Zu- und Abführen von Spülflüssigkeit zu und von der Filtermembran geeignet sind. Die Spülflüssigkeiten wird mittels einer zweiten spülflüssigkeitführenden Anschlussleitung in die gasführbare Zuführleitung eingespeist und dient zum Spülen des gesamten Leitungssystems und der Filtermembran, um das Verkleben und Verstopfen, insbesondere der Abführleitung, durch Inhaltsstoffe der Substanzen der Probe zu vermeiden. Die Spülflüssigkeit ist eine sterile Flüssigkeit. Nach dem Spülvorgang wird das Leitungssystem wiederum mit Gas aus der ersten Anschlußleitung mit geringem Überdruck entleert. Ein derartiger Spülvorgang kann optional zwischen zwei Probenahmevorgängen durchgeführt werden.

Das Verfahren zur Entnahme von flüssigen Proben aus mit dem Medium gefüllten Behältern und/oder Rohren, insbesondere aus Fermentern, über eine Filtermembran (5) mittels Unterdruck weist folgende Schritte auf:
- Zuführen eines Gases zu einer in der Probesonde angeordneten Filtermembran aus dem als Sterilgrenze wirkenden Material auf der sterilen Grenzseite der Filtermembran mittels der mit mindestens einem Ventil sperrbaren Zuführleitung;
- Abführen des Gases von der Filtermembran mittels einer separaten Abführleitung und Öffnen einer in der Abführleitung angeordneten ventilwirkenden Vorrichtung solange, bis die Zu- und Abführleitungen probenfrei sind;
- Schließen mindestens eines Ventils zum Abkoppeln der Zuführleitung von einem Gasversorgungsanschluß;
- Entnahme der Probe mit gewünschtem Volumen aus dem Medium mittels der separaten Abführleitung und einem in der Abführleitung vorhandenen Unterdruck und
- Beförderung der Probe aus der Abführleitung mittels des mit Überdruck erneut zugeführten Gases.

Zusätzlich kann zur Vermeidung von Verstopfungen und Verklebungen der Abführleitung das Verfahren den Schritt der Zuführung von Spülflüssigkeit und für die Durchführung des Integritätstests die Zuführung von Gas unter Abschluß des Vorrichtungsinnenraums mittels Ventilen gegenüber den umliegenden Systemen beinhalten.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: eine schematische Darstellung einer Vorrichtung zur Entnahme von Proben gemäß einer ersten Ausführungsform der Erfindung,
- Fig.2: eine schematische Darstellung eines Ausschnitts der Vorrichtung zur Entnahme von Proben gemäß einer zweiten Ausführungsform der Erfindung;
- Fig. 3: ein Diagramm gemessener Glukosekönzentrationen über einen bestimmten Zeitraum hinweg bei Probenahmevorgängen mit einer vergleichenden Darstellung von Probenahmen mittels einer erfindungsgemäßen Vorrichtung und mittels einer herkömmlichen, kommerziell erhältlichen Vorrichtung, und
- Fig. 4: ein Diagramm gemessener Lactatkonzentrationen über einen bestimmten Zeitraum hinweg bei Probenahmevorgängen mit einer vergleichenden Darstellung von Probenahmen mittels der erfindungsgemäßen Vorrichtung und mittels der herkömmlichen, kommerziell erhältlichen Vorrichtung.

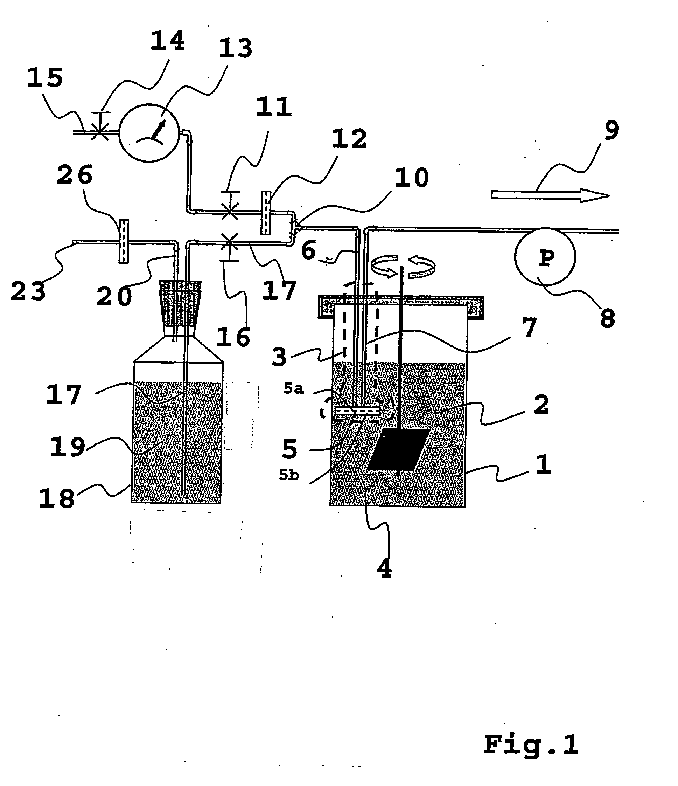

Figur 1 zeigt in einer schematischen Darstellung eine Vorrichtung zur Entnahme von Proben aus einem mit einem Medium gefüllten Behälter gemäß einer Ausführungsform der Erfindung. In das in dem Behälter 1 enthaltene Medium 2 ist eine Probenahmesonde 3 und ein Rührer 4 zum Umrühren des Mediums 2 geführt. Innerhalb der Probenahmesonde 3 ist eine Filtermembran aus einem als Sterilgrenze wirkenden Material angeordnet. Die Filtermembran 5 weist eine sterile Rückgrenzseite 5a und eine zu dem Medium hingewandte Vordergrenzseite 5b auf, wobei das Medium steril oder nicht-steril ausgebildet sein kann.

An der Rückgrenzseite 5a der Filtermembran 5 - also auf der sterilen Grenzseite der Filtermembran - ist eine gasführbare Zuführleitung 6 und eine probeführbare Abführleitung 7 angeordnet. Über die Abführleitung 7 kann eine Probe, die durch einen Unterdruck einer Pumpe 8 durch die Filtermembran 5 hindurch aus dem Medium angesaugt wird, in eine hier nicht gezeigte Detektoreinrichtung oder einen hier nicht gezeigten Vorratsbehälter abgeführt werden, wie es durch den Pfeil 9 angedeutet wird.

Die Zuführleitung 6 ist mittels eines T-Stückes 10 mit einer ersten gasführenden Anschlussleitung verbunden, die wiederum mit einem Gasversorgungsanschluß 15 in Verbindung steht. Über den Gasversorgungsanschluß 15 wird Druckluft der Zuführleitung 6 zugeführt, sofern sperrbare Ventile 11 und 14 in ihrer Öffnungsstellung angeordnet sind.

Um eine Zuführung von steriler Druckluft sicherzustellen, ist ein Sterilfilter 12 angeordnet. Zusätzlich wird die unter leichtem Überdruck zugeführte Druckluft mittels eines Manometers 13 druckgeprüft.

An dem weiteren Ende des T-Stücks 10 ist eine zweite spülflüssigkeitsführende Anschlußleitung 17 angeschlossen, die mit einem Behälter 18 in Verbindung steht, in dem Spülflüssigkeit 19, wie beispielsweise destilliertes Wasser, in ausreichendem Maße vorhanden ist. Eine Gas- und Spülflüssigkeitsanschlussleitung 20 verbindet den Behälter 18 über einen darin angeordneten zweiten Sterilfilter 26 mit einem weiteren Gasversorgungsanschluß 23, um einen Druckausgleich für den Behälter 18 zu schaffen. Dieser Gasversorgungsanschluß 23 ist wahlweise zuschaltbar.

Sofern ein Spülvorgang durchgeführt werden soll, wird ein Ventil 16 zur Zuführleitung 6 hin geöffnet, während die Ventile 11 und 14 der ersten gasführenden Anschlußleitung geschlossen werden. Nachdem die Spülflüssigkeit die Zu- und Abführleitungen 6, 7 über die Pumpe 8 verlassen hat, und eine Spülung der Leitungen 6, 7 und der Rückgrenzseite 5a der Filtermembran durchgeführt worden ist, wird das Ventil 16 geschlossen und die ersten und zweiten Ventile 11, 14 werden geöffnet, um einen Entleerungsvorgang des Leitungssystems mit den Zu- und Abführleitungen 6, 7 mittels der zugeführten sterilen Druckluft zu bewirken.

### Bei einem Probenahmevorgang findet folgender Ablauf statt:

Die Pumpe 8 erzeugt, während das erste und zweite Ventil 11, 14 geschlossen sind, einen Unterdruck innerhalb des Leitungssystems, der eine Probe aus dem zu untersuchenden Medium 2 durch die Sterilgrenze hindurch aus der Probenahmesonde fördert. Das gewünschte Probevolumen wird mittels der Pumpstärke der Pumpe 8 reguliert. Nach Erreichen eines vorbestimmten Probevolumens mit einer vorbestimmten Förderzeit wird die Probe mit zusätzlichem geringen Druckluftüberdruck bei geschlossenem Ventil 16 und offenen Ventilen 11 und 14 aus dem Leitungssystem mit den Leitungen 6, 7 gefördert und in einen hier nicht gezeigten Vorratsbehälter eingespeist. Gleichzeitig bewirkt die zugeführte Druckluft zuverlässig eine Entleerung des Leitungssystems, so dass dieses probefrei ist.

Für einen durchführbaren Integritätstest zur Überprüfung der Funktion der Probenahmevorrichtung wird über den Gasversorgungsanschluß 15 bei geschlossenem Ventil 16 und nicht betriebener Pumpe 8 Druckluft zur Erzeugung eines Überdrucks im Leitungssystem zugeführt. Der Überdruck bleibt nach Schließen des Ventils 14 weiterhin erhalten, sofern die Filtermembran 5, welche die Sterilgrenze bildet, dicht ist. Wenn der Druck hingegen abfällt, deutet dies auf eine undichte Filtermembran 5 hin. Die Vorrichtung ist somit bei Anschluß eines Integritätsmessgerätes validierbar.

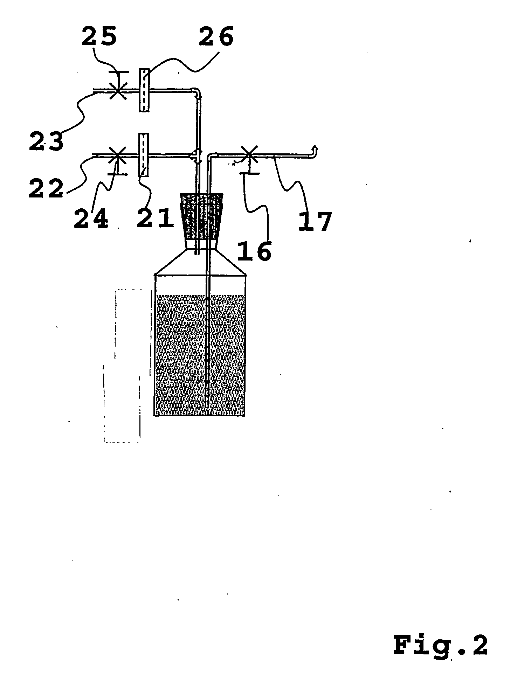

Figur 2 zeigt in einer schematischen Darstellung einen Ausschnitt der Vorrichtung zur Entnahme von Proben gemäß einer zweiten Ausführungsform der Erfindung. In dieser Ausführungsform ist zusätzlich ein Spülflüssigkeitsversorgungsanschluss 22 mit dem Behälter 18 über einen spülflüssigkeitsführenden Teil der Leitung 20 und einem jeweils dazwischen angeordneten weiteren Sterilfilter 21 und weiteren Ventil 24 verbunden. Der Spülflüssigkeitsversorgungsanschluss 22 dient zum Wiederauffüllen des Behälters 18 mit Spülflüssigkeit.
Der gasführende Teil der Leitung 20 weist zusätzlich ein weiteres Ventil 25 zwischen dem Filter 26 und dem Gasversorgungsanschluß 23 zum Zuführen, Abführen oder Dosieren von Gas auf.

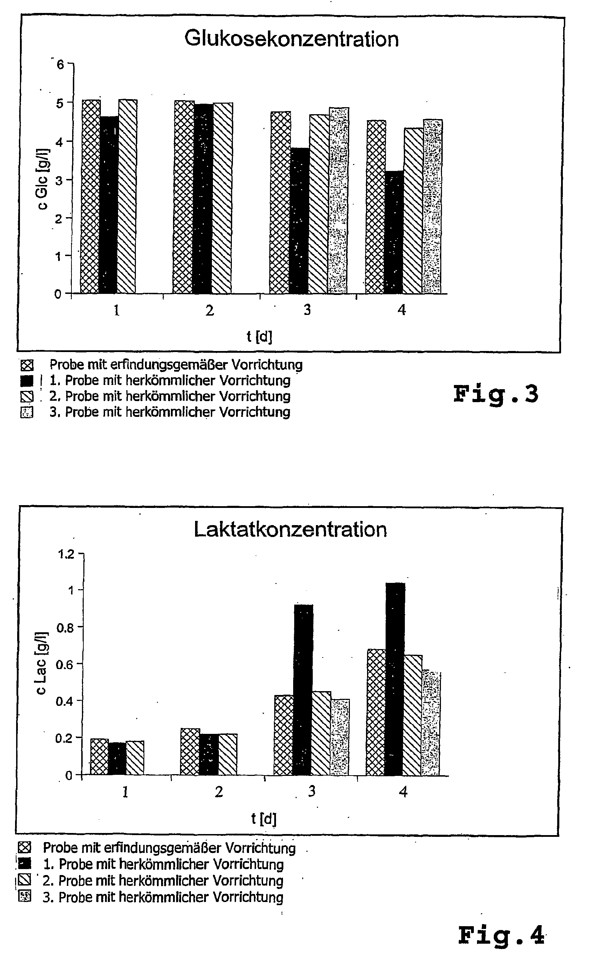

Figur 3 zeigt in einem Diagramm in einer vergleichenden Darstellung eine Glukosekonzentration über einen bestimmten Zeitraum hinweg eine Probeentnahme für einen Probenahmevorgang mittels der erfindungsgemäßen Vorrichtung und mittels einer kommerziell erhältlichen herkömmlichen Vorrichtung.

Es wurden beide Probenahmevorrichtungen in einen Bioreaktor eingebaut. Der Bioreaktor wurde sterilisiert und mit sterilem Medium, Carriern und Zellen befüllt. Die Zellen sollten durch ihr Wachstum die Glukose- und Lactatkonzentration im Medium ändern.

Nach Abschluß aller Vorbereitungen und der Sterilisation wurde bei der erfindungsgemäßen Vorrichtung ein Membrantest durchgeführt. Dann wurde mit beiden Vorrichtungen jeweils eine Probe entnommen und die Glukose- und Lactatkonzentration bestimmt. Bei der herkömmlichen Probenahmevorrichtung musste aufgrund des vorhandenen Totvolumens eine erste Probe verworfen werden. Es wurde das zu verwerfende Probevolumen bis zur Konzentrationskonstanz von Glukose und Lactat ermittelt. In der herkömmlichen Vorrichtung gab es keine ideale Verdrängung der Probenflüssigkeit aus dem Leitungssystem, sondern eine Rückvermischung einzelner Substanzen in der darin enthaltenen Strömung. Die Rückvermischung wurde verursacht durch unterschiedliche Diffusionskoeffizienten der Substanzen in der Probe und zusätzlich durch die fluiddynamischen Eigenschaften von Totvolumen und Probenflüssigkeit.

Die Entnahme der Probe mittels der herkömmlichen Vorrichtung erfolgte nicht kontinuierlich. Das Ventil dieser herkömmlichen Vorrichtung wurde als Drei-Wege-Ventil von einem Strömungsweg zwischen der Probenahmesonde und einem Ausgang auf einen Strömungsweg zwischen einem Überströmanschluß und dem Ausgang umgeschaltet, nachdem bereits 2 ml Probevolumen in das Leitungssystem gefördert wurden.

Dadurch riß der Flüssigkeitsstrom im Inneren der Vorrichtung ab, und es wurde die Durchmischung in der Strömung noch verstärkt. In diesem Fall führte die höhere Durchmischung zur Vergrößerung des benötigten Probevolumens bis zur Konzentrationskonstanz. Aus diesem Grunde wurde für die Zusammenfassung der Absolutwert des entnommenen Gesamtvolumens der herkömmlichen Vorrichtung auf etwa 2,5 ml gesenkt.

### Während der Fermentation ergaben sich folgende Ergebnisse:

Nach der Sterilisation war Wasserdampf im Inneren der herkömmlichen Vorrichtung kondensiert und hatte das Totvolumen der Vorrichtung ausgefüllt. Es war nach dem Einschalten der Pumpe eine deutliche Unterscheidung zwischen dem farblosen Kondensat und dem rotgefärbten Medium möglich. Das aufgefangene und gemessene Totvolumen betrug ca. 0,6 ml. Die Mediumproben wurden in Eppendorf-Tipps gesammelt. Mit der herkömmlichen Vorrichtung mussten mindestens zwei Proben von je 2 ml Probevolumen gesammelt werden, bis eine konstante gemessene Substanzkonzentration eintrat. Dies gilt sowohl für die Glukosekonzentration als auch für die in Figur 4 dargestellte Lactatkonzentration. Dieser Vorgang wurde an den folgenden Tagen wiederholt. Damit ergab sich ein Mehrverbrauch an zu entnehmendem Medium gegenüber dem Probenahmevorgang mittels der erfindungsgemäßen Probenahmevorrichtung zwischen 2 - 4 ml (100 - 200 %).

Aus der nachfolgenden Tabelle 1 läßt sich in vergleichender Darstellung ebenso wie aus den in Figuren 3 und 4 dargestellten Diagrammen die Entnahme von Proben aus dem Bioreaktor bis zum Konzentrationsausgleich von Glukose und Lactat bei einer Entnahme mittels der erfindungsgemäßen und der herkömmlichen Vorrichtung als Messergebnisse entnehmen:

| **Tabelle** 1 | Erfindung | Stand d. Technik | Stand d. Technik | Stand d. Technik |
|---|---|---|---|---|
| *Glucose [g*/*l]* | | | | |
| *t [d]* | *1. Probe* | *1. Probe* | *2. Probe* | *3. Probe* |
| 1 | 5.06 | 4.64 | 5.07 | |
| 2 | 5.03 | 4.95 | 4.99 | |
| 3 | 4.77 | 3.83 | 4.69 | 4.88 |
| 4 | 4.55 | 3.23 | 4.35 | 4.57 |

| *Lactat [g*/*l]* | | | | |
|---|---|---|---|---|
| *t [d]* | | | | |
| 1 | 0,19 | 0.17 | 0.18 | |
| 2 | 0.25 | 0.22 | 0.22 | |
| 3 | 0.43 | 0.92 | 0.45 | 0.41 |
| 4 | 0.68 | 1.04 | 0.65 | 0.57 |

In einem weiteren Versuch wurden aus einer Spinnerflasche mit Casolösung, die einer Nähr bouillon für Steriltests entspricht, mittels der erfindungsgemäßen Vorrichtung mehrfach Proben mit einem Volumen von 1,5 - 2 ml entnommen und analysiert. Über einen Versuchszeitraum von zwei Wochen hinweg war die Vorrichtung steril. Die Konzentration des Mediums innerhalb der Spinnerflasche an Glukose blieb annähernd konstant, abgesehen von Messgeräteschwankungen des Enzymsensors. Diese sind darauf zurückzuführen, dass der Enzymsensor empfindlich auf z. B. alternde Sensormembrane oder ungenügende Kalibrierautomatik reagierte. Aus der nachfolgenden Tabelle 2 ist die Glukosekonzentration, wie sie über mehrere Tage hinweg gemessen wurde, zu entnehmen.

**Tabelle 2**

| ***t [d]*** | ***Konzentration t [d] [g*/*l Glc]*** | ***Konzentration [g*/*l Glc]*** | ***t [d]*** | ***Konzentration [g*/*l Glc]*** |
|---|---|---|---|---|
| 0 | 1,23 6 | 0,67 | 9 | 0,98 |
| 4 | 1,06 7 | 0,96 | 11 | 0,98 |
| 5 | 0,99 8 | 1,05 | 12 | 1,00 |

In einem Vorversuch wurde überprüft, ob die Konzentration an Glukose in einer Casolösung mit der Zeit von allein abnimmt. Dafür wurde eine Casolösung steril bei 37° C inkubiert. Die Analyse der Casolösung zeigt eine Stabilität über den Versuchszeitraum von vier Wochen, wie es der nachfolgenden Tabelle 3 zu entnehmen ist.

**Tabelle 3**

| ***t [d]*** | ***Konzentration [g*/*l Glc]*** | ***t [d]*** | ***Konzentration [g*/*l Glc]*** |
|---|---|---|---|
| 0 | 1,52 | 21 | 1,51 |
| 19 | 1,52 | 27 | 1,52 |

Die Ausführung der Erfindung ist nicht nur auf das beschriebene Beispiel und die hervorgehobenen Aspekte beschränkt, sondern ist im Rahmen der Ansprüche ebenso in einer Vielzahl von Abwandlungen möglich, die im Rahmen fachgemäßen Handelns liegen.

### Bezugszeichenliste

- 1: Behälter
- 2: Medium
- 3: Probenahmesonde
- 4: Rührer
- 5: Filtermembran
- 5a: Rückseite, der Filtermembran
- 5b: Vorderseite der Filtermembran
- 6: Zuführleitung
- 7: Abführleitung
- 8: Pumpe
- 9: Abführrichtung
- 10: T-Stück
- 11, 14, 16, 24, 25: Ventile
- 12, 21, 26: Sterilfilter
- 13: Manometer
- 15, 23: Gasversorgungsanschluß
- 17: spülflüssigkeitführende Anschlußleitung
- 18: Behälter
- 19: Spülflüssigkeit
- 20: Gas- und Spülflüssigkeitsanschlußleitung
- 22: Spülflüssigkeitsversorgungsanschluß
- 23: Gasversorgungsanschluß

## Patentansprüche

1. Vorrichtung zur Entnahme von flüssigen Proben aus mit einem Medium (2) gefüllten Behältern (1) und/oder Rohren, insbesondere aus Fermentern, über eine Filtermembran (5) mittels Unterdruck,
**dadurch gekennzeichnet, dass**
die innerhalb einer Probesonde (3) angeordnete Filtermembran (5) aus einem als Sterilgrenze wirkenden Material besteht, wobei auf der dem Medium (2) abgewandten sterilen Grenzseite (5a) der Filtermembran (5) eine gasführbare Zuführleitung (6) sowie eine separate probeführbare Abführleitung (7) angeordnet sind.

2. Vorrichtung nach Anspruch 1,
**dadurch gekennzeichnet, dass**
das als Sterilgrenze wirkende Material hydrophil ist.

3. Vorrichtung nach Anspruch 1 oder 2,
**dadurch gekennzeichnet, dass**
durch die gasführbare Zuführleitung (6) ein hydrophobes Gas führbar ist.

4. Vorrichtung nach einem der vorangegangenen Ansprüche,
**dadurch gekennzeichnet, dass**
die gasführbare Zuführleitung (6) und die Abführleitung (7) derart ausgebildet sind, dass sie zum Zu- und Abführen von Gas mit Überdruck zu und von der Filtermembran (5) geeignet sind.

5. Vorrichtung nach einem der vorangegangenen Ansprüche,
**dadurch gekennzeichnet, dass**
die gasführbare Zuführleitung (6) an eine erste gasführende Anschlußleitung zum Verbinden der Zuführleitung (6) mit einem Gasversorgungsanschluß (15) angeschlossen ist.

6. Vorrichtung nach Anspruch 5,
**dadurch gekennzeichnet, dass**
ein erstes und zweites Ventil (11, 14) im Bereich des ersten und zweiten Endes der Anschlussleitung angeordnet sind.

7. Vorrichtung nach Anspruch 5 oder 6,
**dadurch gekennzeichnet, dass**
ein Drucksensor (13) in der gasführenden Anschlussleitung angeordnet ist.

8. Vorrichtung nach einem der Ansprüche 5 - 7,
**dadurch gekennzeichnet, dass**
ein erster Sterilfilter (12) in der gasführenden Anschlussleitung angeordnet ist.

9. Vorrichtung nach einem der vorangegangenen Ansprüche,
**dadurch gekennzeichnet, dass**
die Zu- und Abführleitungen (6, 7) derart ausgebildet sind, dass sie zum Zu- und Abführen von Spülflüssigkeiten zu und von der Filtermembran (5) geeignet sind.

10. Vorrichtung nach Anspruch 9,
**dadurch gekennzeichnet, dass**
die Zuführleitung (6) an eine zweite spülflüssigkeitführende Anschlussleitung (17) angeschlossen ist.

11. Vorrichtung nach Anspruch 10,
**dadurch gekennzeichnet, dass**
die spülflüssigkeitführende Anschlussleitung (17) mit einem eine Spülflüssigkeit (19) enthaltenden Behälter (18) verbunden ist.

12. Vorrichtung nach einem der Ansprüche 9 -11,
**dadurch gekennzeichnet, dass**
der Behälter (18) mit einem Spülflüssigkeitsversorgungsanschluß (22) über eine Gasund Spülflüssigkeitsanschlussleitung (20) mit einem darin angeordneten weiteren Sterilfilter (21) verbunden ist.

13. Vorrichtung nach einem der Ansprüche 9 - 12,
**dadurch gekennzeichnet, dass**
der Behälter (18) mit einem weiteren Gasversorgungsanschluß (23) über eine Gasund Spülflüssigkeitsanschlussleitung (20) mit einem darin angeordneten weiteren Sterilfilter (26) verbunden ist.

14. Vorrichtung nach einem der vorangegangenen Ansprüche,
**dadurch gekennzeichnet, dass**
die Abführleitung (7) mit einer ventilwirkenden Vorrichtung (8) verbunden ist.

15. Verfahren zur Entnahme von flüssigen Proben aus mit einem Medium (2) gefüllten Behältern (1) und/oder Rohren, insbesondere aus Fermentern, über eine Filtermembran (5) mittels Unterdruck
**gekennzeichnet durch**
folgende Schritte:
- Zuführen eines Gases zu einer in einer Probesonde angeordneten Filtermembran (5) aus einem als Sterilgrenze wirkenden Material auf der sterilen Grenzseite der Filtermembran (5) mittels einer mit mindestens einem Ventil (11) sperrbaren Zuführleitung (6),
- Abführen des Gases von der Filtermembran (5) mittels einer separaten Abführleitung (7) und Öffnen einer in der Abführleitung (7) angeordneten ventilwirkenden Vorrichtung (8) solange, bis die Zu- und Abführleitungen (6, 7) probenfrei sind,
- Schließen mindestens eines Ventils (11) zum Abkoppeln der Zuführleitung (6) von einem Gasversorgungsanschluß (15),
- Entnahme der Probe mit gewünschten Volumen aus dem Medium (2) mittels der separaten Abführleitung (7) und einem in der Abführleitung (7) vorhandenen Unterdruck, und
- Beförderung der Probe aus der separaten Abführleitung (7) mittels eines mit Überdruck erneut zugeführten Gases.

16. Verfahren nach Anspruch 15,
**dadurch gekennzeichnet, dass**
zur Vermeidung von durch Inhaltsstoffe der Probe verursachte Verstopfungen und Verklebungen innerhalb der Abführleitung (7) eine Spülflüssigkeit (19) nach dem Schritt der Beförderung der Probe aus der Abführleitung (7) über die Zuführleitung (6) zugeführt und über die Abführleitung (7) abgeführt wird.

17. Verfahren nach Anspruch 16,
**dadurch gekennzeichnet, dass**
nach dem Schritt des Zu- und Abführens der Spülflüssigkeit (19) die Schritte des Zuund Abführens des Gases wiederholt werden.

18. Verfahren nach einem der Ansprüche 15 - 17,
**dadurch gekennzeichnet, dass**
ein Integritätstest zur Prüfung/Validierung der Funktion der Probenahme die Schritte umfasst:
- Verschließen der Abführleitung (7) durch eine ventilwirkende Vorrichtung (8),
- Zuführen von Gas in die Zu- und Abführleitungen (6, 7) zur Erzeugung eines definierten Überdruckes,
- Schließen eines weiteren Ventils (14) zur Abkopplung des Gasversorgungsanschlusses (15) von der Zuführleitung (6) unter Miteinbeziehung eines Drucksensors (13),
- Beobachten von möglichen Gas- und/oder Flüssigkeitsein- und/oder -austritten aus dem Leitungssystems, und
- Beobachten der Druckstabilität mittels des Drucksensors (13) als Indikator für die Integrität der Filtermembran (5).

## Claims

1. Device for extracting liquid samples from containers (1) and/or tubes (1) filled with a medium (2), in particular from fermenters, via a filter membrane (5) by means of partial vacuum,
**characterised in that**
the filter membrane (5) arranged within a sample probe (3) comprises a material acting as sterile boundary, wherein on the sterile boundary side (5a) of the filter membrane (5), which faces away from the medium (2), a supply line (6), which can be used to guide gas, and a separate discharge line (7), which can be used to guide the sample, are arranged.

2. Device according to claim 1,
**characterised in that**
the material acting as a sterile boundary is hydrophilic.

3. Device according to either claim 1 or 2,
**characterised in that**
a hydrophobic gas is guidable through the supply line (6), which can be used to guide gas.

4. Device according to any one of the preceding claims,
**characterised in that**
the supply line (6), which can be used to guide gas, and the discharge line (7) are designed to be suitable to supply and discharge gas with overpressure to and from the filter membrane (5).

5. Device according to any one of the preceding claims,
**characterised in that**
the supply line (6), which can be used to guide gas, is connected to a first gas-bearing connecting line to connect the supply line (6) to a gas supply connection (15).

6. Device according to claim 5,
**characterised in that**
a first and second valve (11, 14) are arranged in the area of the first and second end of the connecting line.

7. Device according to either claim 5 or 6,
**characterised in that**
a pressure sensor (13) is arranged in the gas-bearing connecting line.

8. Device according to any one of claims 5-7,
**characterised in that**
a first sterile filter (12) is arranged in the gas-bearing connecting line.

9. Device according to any one of the preceding claims,
**characterised in that**
the supply and discharge lines (6, 7) are designed to be suitable to supply and discharge rinsing liquids to and from the filter membrane (5).

10. Device according to claim 9,
**characterised in that**
the supply line (6) is connected to a second rinsing liquid-bearing connecting line (17).

11. Device according to claim 10,
**characterised in that**
the rinsing liquid-bearing connecting line (17) is connected to a container (18) containing a rinsing liquid (19).

12. Device according to any one of claims 9-11,
**characterised in that**
the container (18) is connected to a rinsing liquid supply connection (22) via a gas and rinsing liquid connecting line (20) with an another sterile filter (21) arranged therein.

13. Device according to any one of claims 9-12,
**characterised in that**
the container (18) is connected to another gas supply connection (23) via a gas and rinsing liquid connecting line (20) with another sterile filter (26) arranged therein

14. Device according to any one of the preceding claims,
**characterised in that**
the discharge line (7) is connected to a device (8) acting as a valve.

15. Method for extracting liquid samples from containers (1) and/or tubes filled with a medium (2), in particular from fermenters, via a filter membrane (5) by means of a partial vacuum
**characterized by**
the following steps:
- supplying of a gas to a filter membrane (5) arranged in the sample probe and made from a material acting as a sterile boundary on the sterile boundary side of the filter membrane (5) by means of a supply line (6) which may be closed by at least one valve (11),
- discharging of the gas from the filter membrane (5) by means of a separate discharge line (7) and opening of a device (8) arranged in the discharge line, which is functioning as a valve until the supply and discharge lines (6, 7) are sample-free.
- closing of at least one valve (11) to uncouple the supply line (6) from a gas supply connection (15).
- extracting of the required volume of the sample from the medium (2) by means of the separate discharge line (7) and a partial vacuum present in the discharge line (7), and
- transporting of the sample out of the separate discharge line (7) by means of gas supplied again by means of overpressure.

16. Method according to claim 15,
**characterised in that**
to avoid clogging and jamming within the discharge line (7) caused by the constituents of the sample, after the step in which the sample is transported out of the discharge line (7), a rinsing liquid (19) is supplied via the supply line (6) and discharged via the discharge line (7).

17. Method according to claim 16,
**characterised in that**
after the step in which the rinsing liquid (19) is supplied and discharged, the steps of supplying and discharging the gas are repeated.

18. Method according to any one of claims 15-17,
**characterised in that**
an integrity test for checking/validating the function of sampling comprises the following steps:
- closing of the discharge line (7) by a device (8) acting as a valve,
- supplying of gas to the supply and discharge lines (6, 7) to generate a defined overpressure,
- closing of another valve (14) to uncouple the gas supply connection (15) from the supply line (6) involvement of a pressure sensor (13),
- observing of any possible gas and/or liquids entering and/or leaving the pipe system, and
- observing of the pressure stability by means of the pressure sensor (13) as an indicator of the integrity of the filter membrane (5).

## Revendications

1. Dispositif de prélèvement des échantillons liquides à partir des récipients (1) et/ou des tubes remplis d'un milieu (2), en particulier des fermenteurs, via une membrane filtrante (5) au moyen d'une dépression, **caractérisé en ce que**
la membrane filtrante (5) agencée à l'intérieur d'une sonde de prélèvement d'échantillon (3) consiste en un matériau agissant en tant que limite stérile, dans lequel un conduit d'acheminement (6) pouvant acheminer un gaz ainsi qu'un conduit d'évacuation (7) séparé pouvant acheminer un échantillon sont agencés sur le côté de limite (5a) stérile, opposé au milieu (2), de la membrane filtrante (5).

2. Dispositif selon la revendication 1,
**caractérisé en ce que**
le matériau agissant en tant que limite stérile est hydrophile.

3. Dispositif selon la revendication 1 ou 2,
**caractérisé en ce que**
un gaz hydrophobe peut être acheminé par le conduit d'acheminement (6) pouvant acheminer un gaz.

4. Dispositif selon l'une quelconque des revendications précédentes,
**caractérisé en ce que**
le conduit d'acheminement (6) pouvant acheminer un gaz et le conduit d'évacuation (7) sont formés de telle sorte qu'ils conviennent à l'acheminement et à l'évacuation d'un gaz avec une surpression vers et depuis la membrane filtrante (5).

5. Dispositif selon l'une quelconque des revendications précédentes,
**caractérisé en ce que**
le conduit d'acheminement (6) pouvant acheminer un gaz est raccordé à un premier conduit de raccordement acheminant un gaz pour une jonction du raccordement du conduit d'acheminement (6) et d'un raccord d'alimentation en gaz (15).

6. Dispositif selon la revendication 5,
**caractérisé en ce que**
une première et une seconde soupapes (11, 14) sont agencées dans la zone des première et seconde extrémités du conduit de raccordement.

7. Dispositif selon la revendication 5 ou 6,
**caractérisé en ce que**
un capteur de pression (13) est agencé dans le conduit de raccordement acheminant un gaz.

8. Dispositif selon l'une quelconque des revendications 5 à 7,
**caractérisé en ce que**
un premier filtre stérile (12) est agencé dans le conduit de raccordement acheminant un gaz.

9. Dispositif selon l'une quelconque des revendications précédentes, ca
ractérisé en ce que
les conduits d'acheminement et d'évacuation (6, 7) sont formés de telle sorte qu'ils conviennent à l'acheminement et à l'évacuation de liquides de lavage vers et depuis la membrane filtrante (5).

10. Dispositif selon la revendication 9,
**caractérisé en ce que**
le conduit d'acheminement (6) est raccordé à un second conduit de raccordement (17) acheminant un liquide de lavage.

11. Dispositif selon la revendication 10,
**caractérisé en ce que**
le conduit de raccordement (17) acheminant un liquide de lavage est relié à un récipient (18) contenant un liquide de lavage (19).

12. Dispositif selon l'une quelconque des revendications 9 à 11, ca
ractérisé en ce que
le récipient (18) est relié à un raccord d'alimentation en liquide de lavage (22) via un conduit de raccordement de gaz et de liquide de lavage (20) avec un autre filtre stérile (21) qui y est agencé.

13. Dispositif selon l'une quelconque des revendications 9 à 12, ca
ractérisé en ce que
le récipient (18) est relié à un autre raccord d'alimentation en gaz (23) via un conduit de raccordement de gaz et de liquide de lavage (20) avec un autre filtre stérile (26) qui y est agencé.

14. Dispositif selon l'une quelconque des revendications précédentes, ca
ractérisé en ce que
le conduit d'évacuation (7) est relié à un dispositif agissant en soupape (8).

15. Procède de prélèvement des échantillons liquides à partir des récipients (1) et/ou des tubes remplis d'un milieu (2), en particulier des fermenteurs, via une membrane filtrante (5) au moyen d'une dépression, **caractérisé par** les étapes suivantes consistant à :
- acheminer un gaz jusqu'à une membrane filtrante (5) agencée dans une sonde de prélèvement d'échantillon, en provenance d'un matériau agissant comme limite stérile sur le côté de limite stérile de la membrane filtrante (5) au moyen d'un conduit d'acheminement (6) blocable par au moins une soupape (11),
- évacuer le gaz à partir de la membrane filtrante (5) au moyen d'un conduit d'évacuation (7) séparé et ouvrir un dispositif agissant comme soupape (8) et agencé dans le conduit d'évacuation (7) jusqu'à ce que les conduits d'acheminement et d'évacuation (6, 7) soient exempts des échantillons,
- fermer au moins une soupape (11) pour détacher le conduit d'acheminement (6) d'un raccord d'alimentation en gaz (15),
- prélever l'échantillon, dans le volume souhaité, hors du milieu (2) au moyen du conduit d'évacuation (7) séparé et d'une dépression existant dans le conduit d'évacuation (7), et
- transporler l'échantillon en provenance du conduit d'évacuation (7) séparé au moyen d'un gaz réacheminé par une surpression.

16. Procédé selon la revendication 15,
**caractérisé en ce que**,
pour éviter des obstructions et des agglutinations provoquées par des composants de l'échantillon à l'intérieur du conduit d'évacuation (7), après l'étape du transport de l'échantillon en provenance du conduit d'évacuation (7), un liquide de lavage (19) est acheminé via le conduit d'acheminement (6) et évacué via le conduit d'évacuation (7).

17. Procédé selon la revendication 16,
**caractérisé en ce que**,
après l'étape d'acheminement et d'évacuation du liquide de lavage (19), on répète les étapes d'acheminement et d'évacuation du gaz.

18. Procédé selon l'une quelconque des revendications 15 à 17, car
actérisé en ce que
un test d'intégrité pour vérifier/valider le fonctionnement du prélèvement d'échantillons comprend les étapes consistant à :
- fermer le conduit d'évacuation (7) à l'aide d'un dispositif agissant en soupape (8),
- acheminer du gaz jusque dans les conduits d'acheminement et d'évacuation (6, 7) pour produire une surpression définie,
- fermer une autre soupape (14) pour détacher le raccord d'alimentation en gaz (15) du conduit d'acheminement (6) tout en y intégrant un capteur de pression (13),
- surveiller des entrées et/ou sorties de gaz et/ou de liquide éventuelles à partir du système des conduits, et
- surveiller la stabilité de pression au moyen du capteur de pression (13) en tant qu'indicateur pour l'intégrité de la membrane filtrante (5).
